# EUROPEAN PATENT APPLICATION

(11) **EP 2 952 210 A2**
(43) Date of publication of application: **09.12.2015**
(21) Application number: 15161756.0
(22) Date of filing: 04.04.2012
(51) Int. Cl.: A61K 47/48, A61K 47/30, A61K 39/395, A61P 35/00

(54) **COMPOSITIONS COMPRISING SACCHARIDE BINDING MOIETIES AND METHODS FOR TARGETED THERAPY**

(30) Priority: 07.04.2011 US 201161472797 P
(62) Divisional of application: 12767424.0
(71) Applicant: Emory University, Atlanta, GA 30322 (US); Georgia Tech Research Corporation, Atlanta, Georgia 30332-0415 (US)
(72) Inventor: Nash, Rodney, J., Atlanta, Georgia 30322 (US); Chappa, Prasanthi, Atlanta, Georgia 30329 (US); Hadjipanayis, Constantinos, G., Decatur, Georgia 30033 (US); Wilson, David, Scott, Broken Arrow, Oklahoma 74012 (US)
(74) Representative: Lau, Sarah Jane

(57) **Abstract**

The disclosure relates to uses of saccharide binding moieties, e.g., lectins for targeting cells, typically cancer stem cells. In certain embodiments, the disclosure relates to conjugates comprising: a) a saccharide binding moiety; b) a polymer; and c) a therapeutic agent; wherein the saccharide binding protein is covalently attached to the polymer.

SEQUENCE LISTING

<110>  Emory University
       Wilson, David S.
       Nash, Rodney J.
       Chappa, Prasanthi
       Hadjipanayis, Constantinos G.

<120>  COMPOSITIONS COMPRISING SACCHARIDE BINDING MOIETIES AND METHODS
       FOR TARGETED THERAPY

<130>  11094PCT

<150>  US 61/472797
<151>  2011-04-07

<160>  7

<170>  PatentIn version 3.5

<210>  1
<211>  19
<212>  PRT
<213>  Homo sapiens

<400>  1

Thr Phe Phe Tyr Gly Gly Ser Arg Gly Lys Arg Asn Asn Phe Lys Thr
1               5                   10                  15

Glu Glu Tyr

<210>  2
<211>  252
<212>  PRT
<213>  Homo sapiens

<400>  2

Asp Ile Gln Ser Phe Ser Phe Lys Asn Phe Asn Ser Ser Ser Phe Ile
1               5                   10                  15

Leu Gln Gly Asp Ala Thr Val Ser Ser Ser Lys Leu Arg Leu Thr Lys
            20                  25                  30

Val Lys Gly Asn Gly Leu Pro Thr Leu Ser Ser Leu Gly Arg Ala Phe
        35                  40                  45

Tyr Ser Ser Pro Ile Gln Ile Tyr Asp Lys Ser Thr Gly Ala Val Ala
    50                  55                  60

Ser Trp Ala Thr Ser Phe Thr Ala Asn Ile Phe Ala Pro Asn Lys Ser
65                  70                  75                  80

Ser Ser Ala Asp Gly Ile Ala Phe Ala Leu Val Pro Val Gly Ser Glu

85                  90                  95

Pro Lys Ser Asn Ser Gly Phe Leu Gly Val Phe Asp Ser Asp Val Tyr
            100                 105                 110

Asp Asn Ser Ala Gln Thr Val Ala Val Glu Phe Asp Thr Phe Ser Asn
        115                 120                 125

Thr Asp Trp Asp Pro Thr Ser Arg His Ile Gly Ile Asp Val Asn Ser
    130                 135                 140

Ile Lys Ser Ile Arg Thr Ala Ser Trp Gly Leu Ala Asn Gly Gln Asn
145                 150                 155                 160

Ala Glu Ile Leu Ile Thr Tyr Asn Ala Ala Thr Ser Leu Leu Val Ala
                165                 170                 175

Ser Leu Val His Pro Ser Arg Arg Thr Ser Tyr Ile Val Ser Glu Arg
            180                 185                 190

Val Asp Ile Thr Asn Glu Leu Pro Glu Tyr Val Ser Ile Gly Phe Ser
        195                 200                 205

Ala Thr Thr Gly Leu Ser Glu Gly Tyr Thr Glu Thr His Asp Val Leu
    210                 215                 220

Ser Trp Ser Phe Ala Ser Lys Leu Pro Asp Asp Ser Thr Thr Glu Pro
225                 230                 235                 240

Leu Asp Ile Ala Ser Tyr Leu Val Arg Asn Val Leu
                245                 250

<210>  3
<211>  273
<212>  PRT
<213>  Homo sapiens

<400>  3

Met Lys Pro Phe Cys Val Phe Leu Thr Phe Phe Leu Leu Leu Ala Ala
1               5                   10                  15

Ser Ser Lys Lys Val Asp Ser Ala Glu Thr Val Ser Phe Asn Phe Asn
            20                  25                  30

Ser Phe Ser Glu Gly Asn Pro Ala Ile Asn Phe Gln Gly Asp Val Thr
        35                  40                  45

Val Leu Ser Asn Gly Asn Ile Gln Leu Thr Asn Leu Asn Lys Val Asn

50                  55                  60

Ser Val Gly Arg Val Leu Tyr Ala Met Pro Val Arg Ile Trp Ser Ser
65                  70                  75                  80

Ala Thr Gly Asn Val Ala Ser Phe Leu Thr Ser Phe Ser Phe Glu Met
                85                  90                  95

Lys Asp Ile Lys Asp Tyr Asp Pro Ala Asp Gly Ile Ile Phe Phe Ile
            100                 105                 110

Ala Pro Glu Asp Thr Gln Ile Pro Ala Gly Ser Ile Gly Gly Gly Thr
        115                 120                 125

Leu Gly Val Ser Asp Thr Lys Gly Ala Gly His Phe Val Gly Val Glu
    130                 135                 140

Phe Asp Thr Tyr Ser Asn Ser Glu Tyr Asn Asp Pro Pro Thr Asp His
145                 150                 155                 160

Val Gly Ile Asp Val Asn Ser Val Asp Ser Val Lys Thr Val Pro Trp
                165                 170                 175

Asn Ser Val Ser Gly Ala Val Val Lys Val Thr Val Ile Tyr Asp Ser
            180                 185                 190

Ser Thr Lys Thr Leu Ser Val Ala Val Thr Asn Asp Asn Gly Asp Ile
        195                 200                 205

Thr Thr Ile Ala Gln Val Val Asp Leu Lys Ala Lys Leu Pro Glu Arg
    210                 215                 220

Val Lys Phe Gly Phe Ser Ala Ser Gly Ser Leu Gly Gly Arg Gln Ile
225                 230                 235                 240

His Leu Ile Arg Ser Trp Ser Phe Thr Ser Thr Leu Ile Thr Thr Thr
                245                 250                 255

Arg Arg Ser Ile Asp Asn Asn Glu Lys Lys Ile Met Asn Met Ala Ser
            260                 265                 270

Ala

<210>  4
<211>  253
<212>  PRT
<213>  Homo sapiens

<400>  4

Ala Glu Thr Val Ser Phe Ser Trp Asn Lys Phe Val Pro Lys Gln Pro
1               5                   10                  15

Asn Met Ile Leu Gln Gly Asp Ala Ile Val Thr Ser Ser Gly Lys Leu
            20                  25                  30

Gln Leu Asn Lys Val Asp Glu Asn Gly Thr Pro Lys Pro Ser Ser Leu
        35                  40                  45

Gly Arg Ala Leu Tyr Ser Thr Pro Ile His Ile Trp Asp Lys Glu Thr
    50                  55                  60

Gly Ser Val Ala Ser Phe Ala Ala Ser Phe Asn Phe Thr Phe Tyr Ala
65                  70                  75                  80

Pro Asp Thr Lys Arg Leu Ala Asp Gly Leu Ala Phe Phe Leu Ala Pro
                85                  90                  95

Ile Asp Thr Lys Pro Gln Thr His Ala Gly Tyr Leu Gly Leu Phe Asn
            100                 105                 110

Glu Asn Glu Ser Gly Asp Gln Val Val Ala Val Glu Phe Asp Thr Phe
        115                 120                 125

Arg Asn Ser Trp Asp Pro Pro Asn Pro His Ile Gly Ile Asn Val Asn
    130                 135                 140

Ser Ile Arg Ser Ile Lys Thr Thr Ser Trp Asp Leu Ala Asn Asn Lys
145                 150                 155                 160

Val Ala Lys Val Leu Ile Thr Tyr Asp Ala Ser Thr Ser Leu Leu Val
                165                 170                 175

Ala Ser Leu Val Tyr Pro Ser Gln Arg Thr Ser Asn Ile Leu Ser Asp
            180                 185                 190

Val Val Asp Leu Lys Thr Ser Leu Pro Glu Trp Val Arg Ile Gly Phe
        195                 200                 205

Ser Ala Ala Thr Gly Leu Asp Ile Pro Gly Glu Ser His Asp Val Leu
    210                 215                 220

Ser Trp Ser Phe Ala Ser Asn Leu Pro His Ala Ser Ser Asn Ile Asp
225                 230                 235                 240

Pro Leu Asp Leu Thr Ser Phe Val Leu His Glu Ala Ile
                245                 250

<210>  5
<211>  251
<212>  PRT
<213>  Homo sapiens

<400>  5

Phe Asn Leu Pro Asn Phe Trp Ser Asp Val Lys Asp Asn Ile Ile Phe
1               5                   10                  15

Gln Gly Asp Ala Asn Thr Thr Ala Gly Thr Leu Gln Leu Cys Lys Thr
            20                  25                  30

Asn Gln Tyr Gly Asn Pro Leu Gln Tyr Arg Ala Gly Arg Ala Leu Tyr
        35                  40                  45

Ser Asp Pro Val Gln Leu Trp Asp Asn Lys Thr Gly Ser Val Ala Ser
    50                  55                  60

Phe Tyr Thr Glu Phe Thr Phe Phe Leu Lys Ile Thr Gly Asp Gly Pro
65                  70                  75                  80

Ala Asp Gly Leu Ala Phe Phe Leu Ala Pro Pro Asp Ser Asp Val Lys
                85                  90                  95

Asp Ala Gly Ala Tyr Leu Gly Leu Phe Asn Lys Ser Thr Ala Thr Gln
            100                 105                 110

Pro Ser Lys Asn Gln Val Val Ala Val Glu Phe Asp Thr Trp Lys Asn
        115                 120                 125

Thr Asp Phe Pro Glu Pro Ser Tyr Arg His Ile Gly Ile Asn Val Asn
    130                 135                 140

Ser Ile Val Ser Val Ala Thr Lys Arg Trp Glu Asp Ser Asp Ile Phe
145                 150                 155                 160

Ser Gly Lys Ile Ala Thr Ala Arg Ile Ser Tyr Asp Gly Ser Ala Lys
                165                 170                 175

Ile Leu Thr Val Val Leu Ser Tyr Pro Asp Gly Ala Asp Tyr Ile Leu
            180                 185                 190

Ser His Ser Val Asp Leu Ser Lys Asn Leu Pro Asn Pro Ile Arg Val
        195                 200                 205

Gly Ile Ser Ala Ser Thr Gly Ala Asn Gln Phe Leu Thr Val Tyr Val
    210                 215                 220

Leu Ser Trp Arg Phe Ser Ser Ala Leu Gln Ser Thr Ser Val Asn Ala
225                 230                 235                 240

Ala Met Gly Pro Glu Ile Ile Arg Thr Val Val
                245                 250

<210>  6
<211>  51
<212>  PRT
<213>  Homo sapiens

<400>  6

Met Ala Lys Ser Thr Ala Lys Pro Asn Phe Ser Leu Leu Leu Pro Ile
1               5                   10                  15

Leu Ile Ser Leu Phe Leu Phe Gln Leu Asn Arg Val Lys Ser Ala Asp
            20                  25                  30

Thr Val Cys Phe Thr Ser Thr Ser Phe Gly Lys Asp Val Ser Asp Leu
        35                  40                  45

Thr Leu Gln
    50

<210>  7
<211>  242
<212>  PRT
<213>  Homo sapiens

<400>  7

Val Glu Thr Ile Ser Phe Ser Phe Ser Glu Phe Glu Pro Gly Asn Asn
1               5                   10                  15

Asp Leu Thr Leu Gln Gly Ala Ala Ile Ile Thr Gln Ser Gly Val Leu
            20                  25                  30

Gln Leu Thr Lys Ile Asn Gln Asn Gly Met Pro Ala Trp Asp Ser Thr
        35                  40                  45

Gly Arg Thr Leu Tyr Thr Lys Pro Val His Ile Trp Asp Met Thr Thr
    50                  55                  60

Gly Thr Val Ala Ser Phe Glu Thr Arg Phe Ser Phe Ser Ile Glu Gln
65                  70                  75                  80

Pro Tyr Thr Arg Pro Leu Pro Ala Asp Gly Leu Val Phe Phe Met Gly

                85                  90                  95

Pro Thr Lys Ser Lys Pro Ala Gln Gly Tyr Gly Tyr Leu Gly Val Phe
            100                 105                 110

Asn Asn Ser Lys Gln Asp Asn Ser Tyr Gln Thr Leu Ala Val Glu Phe
        115                 120                 125

Asp Thr Phe Ser Asn Pro Trp Asp Pro Pro Gln Val Pro His Ile Gly
    130                 135                 140

Ile Asp Val Asn Ser Ile Arg Ser Ile Lys Thr Gln Pro Phe Gln Leu
145                 150                 155                 160

Asp Asn Gly Gln Val Ala Asn Val Val Ile Lys Tyr Asp Ala Ser Ser
                165                 170                 175

Lys Ile Leu Leu Ala Val Leu Val Tyr Pro Ser Ser Gly Ala Ile Tyr
            180                 185                 190

Thr Ile Ala Glu Ile Val Asp Val Lys Gln Val Leu Pro Glu Trp Val
        195                 200                 205

Asp Val Gly Leu Ser Gly Ala Thr Gly Ala Gln Arg Asp Ala Ala Glu
    210                 215                 220

Thr His Asp Val Tyr Ser Trp Ser Phe His Ala Ser Leu Pro Glu Thr
225                 230                 235                 240

Asn Asp

## Description

### CROSS REFERENCE TO RELATED APPLICATIONS

This application claims priority to U. S. Provisional Application Number 61/472,797 filed April 7, 2011, hereby incorporated by reference in its entirety.

### BACKGROUND

Current treatment for glioblastoma multiforme (GBM), the most common type of primary brain tumors, remains suboptimal. The treatment can involve chemotherapy, radiotherapy, and surgery, all of which typically do not provide a cure. Surgical removal of tumors often results in re-growth, and using traditionally chemotherapy regiments for treating GBM is believed to be thwarted by the blood-brain barrier. Thus, there is a need for identifying methods preventing tumor growth and improving circulation of chemotherapeutic agents in the brain.

Cancer stem cells (CSCs) are cancer cells that possess some characteristics associated with normal stem cells such as the ability to differentiate; however, CSCs are tumorigenic. If cancer treatments of do not properly destroy enough CSCs after surgery, the tumor will reappear. There is a risk that post-operative chemotherapy will leave only chemotherapy-resistant CSCs and the ensuing tumor will be resistant to future chemotherapy. It becomes more and more difficult to remove the tumor without conferring resistance and leaving CSCs behind for the tumor to reappear. Thus, it is one object of the disclosure to identify compositions and methods for improving the identification and removal of GBM CSCs after surgical intervention.

Since the expression of CD133 is a marker of tumor cells with stem-cell ability, agents aimed at targeting CD133⁺ tumor cells have been disclosed. For example, Smith et al., Br J Cancer, 2008, 99:100-9, disclose CD133 antibody-drug conjugates for use in hepatocellular and gastric cancers. Chearwae & Bright, Br J Cancer, 2008, 99, 2044-2053, disclose that PPARγ agonists inhibit growth and expansion of CD133⁺ brain tumor stem cells.

GBM brain tumor stem cells have been identified through their expression of CD 133; however CD133 is also a marker expressed in non-malignant neural progenitor cells. Using CD133 exclusively as a marker for GBM tumor derived CSCs (GBM-CSCs) is problematic because it is not consistently expressed in all GBMs and CD133-negative cells have been shown to give rise to tumors in transplant assays. See Lathia et al., Stem Cell Rev., 2010, 7(2) 227-237.

The lectin dolichos biflorus agglutinin (DBA), which recognizes α-N-acetylgalactosamine (GalNAc), was used to characterize mouse embryonic stem cells (mESC) cultures by nondestructive measures. Nash et al., Stem Cells, 2007, 25, 974-982. Presentation of GalNAc on the mESC surface is rapidly down-regulated during differentiation preceding that of know protein markers of pluripotency. Lectins have also been used to investigate metastatic processes in cancer as well as to document the repertoire of glycoepitopes on the surface of embryonal carcinoma cells. See, e.g., Venable et al., BMC Dev Biol., 2005, 5, 15 and Plattner et al., Eur J Pharm Biopharm., 2008, 70, 572-576.

Xin et al., International Journal of Pharmaceutics, 2010, 402, 238-247 disclose an antitumor effect of paclitaxel (PTX)-loaded methoxy poly(ethylene glycol)-poly(ε-caprolactone) nanoparticles (MPEG-NP/PTX) against glioblastoma multiforme (GBM). Xin et al., Biomaterials , 2011, 1-13, have also disclosed peptide-conjugated poly(ethylene glycol)-co-poly(e-caprolactone) nanoparticles act as a targeting drug delivery system for brain glioma.

References cited herein are not an admission of prior art.

### SUMMARY

The disclosure relates to uses of saccharide binding moieties and proteins, e.g., lectins for targeting cells, typically cancer stem cells. In certain embodiments, the disclosure contemplates conjugates comprising a saccharide binding moiety and a therapeutic agent optionally containing a biodegradable linker. In certain embodiments, the disclosure relates to conjugates comprising: a) a saccharide binding moiety such as a protein; b) a polymer; and c) a therapeutic agent; wherein the saccharide binding moiety is covalently attached to the polymer. The saccharide binding moiety may be a lectin selected from the group consisting of concanavalin A (CON A), dolichos biflorus agglutinin (DBA), peanut agglutinin (PNA), ricinus communis agglutinin I (RCA 120), soybean agglutinin (SBA), ulex europaeus agglutinin I (UEA I), wheat germ agglutinin (WGA), griffonia simplicifolia lectin I (GSL I), lens culinaris agglutinin (LCA), phaseolus vulgaris erythroagglutinin (PHA-E), phaseolus vulgaris leucoagglutinin (PHA-L), pisum sativum agglutinin (PSA), griffonia simplicifolia lectin II (GSL II), datura stramonium lectin (DSL), erythrina cristagalli lectin (ECL), Jacalin, lycopersicon esculentum lectin (LEL), solanum tuberosum lectin (STL), and vicia villosa lectin (WA) or variant, portions, or derivatives thereof provided the saccharide binding protein binds a saccharide or polysaccharide selected from the group consisting of α- or β-linked N-acetylgalactosamine, branched and terminal α-linked mannose, α-linked N-acetylgalactosamine, galactosyl (β-1,3) N-acetylgalactosamine, oligosaccharides ending in galactose, terminal α- or β-linked N-acetylgalactosamine, α-linked fucose residues, terminal N-acetylglucosamine or chitobiose, α-N-acetylgalactosamine and α-galactose residues, α-linked mannose residues, galactose, α-linked mannose residues, α- or β-linked N-acetylglucosamine, N-acetyllactosamine, galactosyl (β-1,4) N-acetylglucosamine, galactosyl (P -1,3) N-acetylgalactosamine, N-acetylglucosamine, N-acetylglucosamine and N-acetylmuramic acid. Other saccharide binding moieties to these specific saccharides are also contemplated.

In certain embodiments, the polymer forms a particle comprising a biodegradable barrier. In certain embodiments, the polymer comprises lactone containing monomers and ethylene glycol containing monomers. In certain embodiments, the therapeutic agent is an anticancer agent.

In certain embodiments, polymer surrounds the therapeutic agent, or the therapeutic agent is conjugated to the polymer through a biodegradable bond.

In certain embodiments, the therapeutic agent may be cetuximab, temozolomide, bevacizumab, doxorubicin, hydroxydaunorubicin, bleomycin, dactinomycin, vinblastine, dacarbazine, mechlorethamine, cyclophosphamide, etoposide, teniposide, vincristine, prednisone, a platinum agent (e.g., cisplatin, carboplatin, oxaliplatin), fluorouracil, folinic acid, carmustine, rituximab, methotrexate, procarbazine, epirubicin, irinotecan, ifosfamide, chlorambucil, lomustine, leucovorin, fludarabine, thalidomide, dexamethasone, docetaxel, anastrozole, topotecan, combretastatin, or combretastatin A-4 phosphate. In certain embodiments, the therapeutic agent stimulates the nuclear receptor peroxisome proliferator-activated receptor gamma (PPAR-γ) such as pioglitazone and rosiglitazone.

In certain embodiments, the conjugate further comprises a molecule that facilitated transfer across the blood brain barrier, e.g., a ligand of low-density lipoprotein receptor-related protein wherein the ligand is attached to the polymer. In certain embodiments, the ligand of low-density lipoprotein receptor-related protein is a polypeptide of TFFYGGSRGKRNNFKTEEY (SEQ ID NO:1).

In certain embodiments, the disclosure relates to compositions comprising conjugates comprising a saccharide binding moiety or protein and a polymer wherein the saccharide binding moiety or protein is covalently attached to the polymer wherein the polymer surrounds a metal particle.

In certain embodiments, the disclosure relates to compositions comprising conjugates comprising: a) a saccharide binding moiety or protein; b) a polymer; and c) a therapeutic agent; wherein the saccharide binding moiety or protein is covalently attached to the polymer wherein the polymer surrounds a particle. In certain embodiments, the particle has a diameter of between about 200 and 5 nm. In certain embodiments, the particle is metal particle, e.g., an iron oxide particle, elemental iron coated with iron oxide, gold particle, silver particle, a quantum dot, or bismuth encapsulated in a phospholipid core. In certain embodiments, the therapeutic agent is an EGFR antibody such as cetuximab or a HER-2 antibody such as trastuzumab and the saccharide binding protein is DBA.

In certain embodiments, the disclosure relates to methods of treating cancer comprising administering a composition comprising a conjugate disclosed herein to a subject diagnosed with cancer. Typically, the subject is diagnosed with a brain tumor and has previously undergone surgical removal of a brain tumor or radiation therapy. It is contemplated that administered of the composition may be in combination with radiation therapy or the composition is administered orally, by injection, convection enhance delivery, or intracerebrally.

In certain embodiments, the disclosure relates to methods of purifying cells comprising mixing lectins disclosed herein with cells under conditions that an isolated composition of cells is formed.

In certain embodiments, the disclosure relates to methods of detecting cancer stem cells by assaying a sample of cells using lectins disclosed herein. It is contemplated that lectins may be mixed with a sample comprising cells and the binding of certain lectins are measured to indicate the presence or absence of caner stem cells. This information is then reported to the subject and correlated to an appropriate therapeutic strategy, e.g., drug regiment, surgery, or radiotherapy.

### BRIEF DESCRIPTION OF THE FIGURES

Figure 1A shows a schematic representation of CD 133 positive cell sorting.
Figure 1B shows dispersal and culturing of CD133 positive sorted cells.
Figure 1C shows differentiation of CD133 positive cells.
Figure 1D schematically illustrates CD133.
Figure 2A shows data on the differentiation of CD133 positive cells.
Figure 2B shows data on a dated population of lectin labeled CD133 positive cells at day 1 and day 12.
Figure 2C shows data on the topographical display of lectin labeled CD133 positive cells at day 1 and day 12.
Figure 3A shows a population of lectin labeled CD133 positive cells at day 1 and day 12.
Figure 3B shows data on the topographical display of lectin labeled CD133 positive cells at day 1 and day 12.
Figure 3C shows data on the CD133 glycosylation, OCT 4, and Sox 2 expression at day 1 and day 12 in differentiating CD133 cells.
Figure 4A shows data on cell sorting of the parent cell population (PC), DBA positive population (SC-DBA), DBA negative population (SC-Neg), and Dual labeled (DBA and Actin positive) population.
Figure 4B shows a bar graph representation of Figure 4A.
Figure 4C shows data on competing sugars (200mM GalNAc) showing specificity of DBA for GalNAc.
Figure 5A is a schematic illustration of DBA positive cell sorting.
Figure 5B shows data on DBA positive and DBA negative cell grown in culture.
Figure 6 shows data on DBA positive and negative neurosphere differentiation and GFAP labeling. Neurospheres derived from DBA positive and negative sorted cells. After day 12 of differentiation cells were fluorescently labeled for GFAP.
Figure 7 shows data on the quantitation of lectin binding on DBA positive and DBA negative GBM-CSCs (day 1 and day 12) surfaces with 8 different lectins and 3 stem cells markers. The percent of cells with specific carbohydrate expression as determined by flow cytometry using 8 different lectins. The data are means ⁺/- SD of 3 independent assays of CTB-1 GBM-CSCs. CTB-1 cell line was stained with CD 133, OCT4, and SOX2 antibody in addition to lectin staining. Statistical significance (p ≤ 0.05) is indicated by asterisk.
Figure 8A shows data on cytometric representation of individual populations of DBA, CD133 and CD133-G labeled cells analyzed through flow cytometry.
Figure 8B shows data on DBA positive sorted cells (day 1 and day 12 of differentiation) labeled for DBA, CD133, and CD133G, analyzed through flow cytometry.
Figures 9A and 9B show data on GBM-CSCs at day 1 and day 12 of differentiation labeled for Ki67 and cleaved caspase-3. Bar graph representation of DBA positive and DBA negative sorted cells were grown as neurospheres and differentiated on laminin coated plates for 12 days. Cells were labeled at day 1 and day 12 of differentiation for Ki67 (proliferation) and cleaved caspase-3 (apoptosis) and analyzed using flow cytometry.

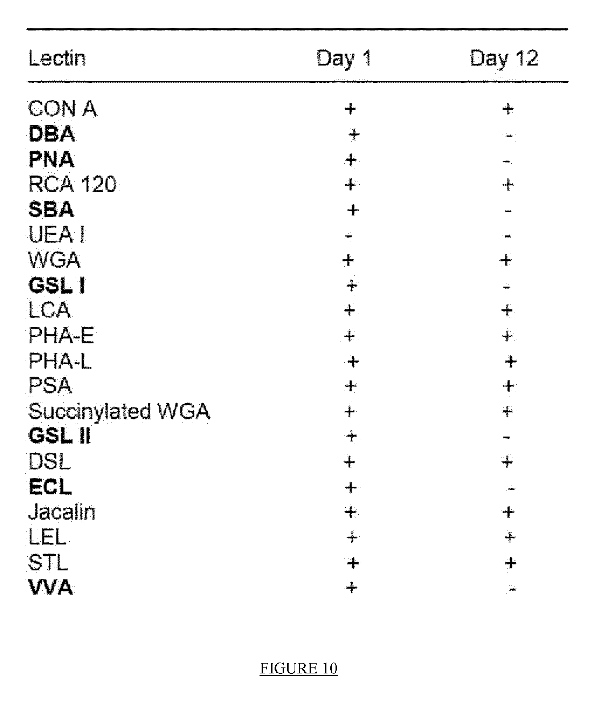

Figure 10 shows a table summarizing data obtained using a panel of 20 fluoresceinisothiocyanate labeled lectins on unfixed (repeated on fixed with the same result) hGBM-CSCs (CTB-1), grown on laminin in CTB-1 neuronal media at day 1 and day 12 in the absence of growth factors.

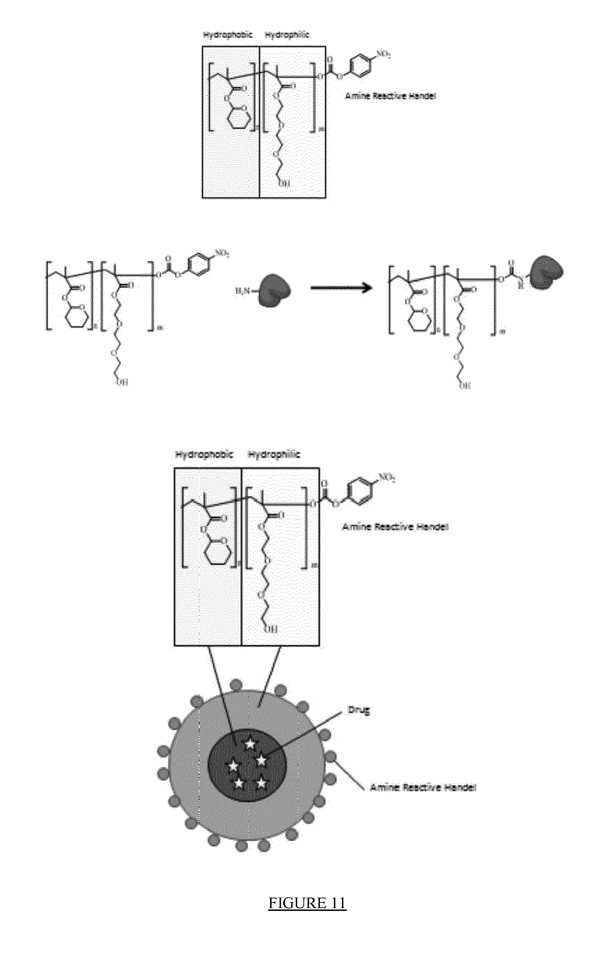

Figure 11 illustrates a lectin conjugated to a particle contain a therapeutic agent.

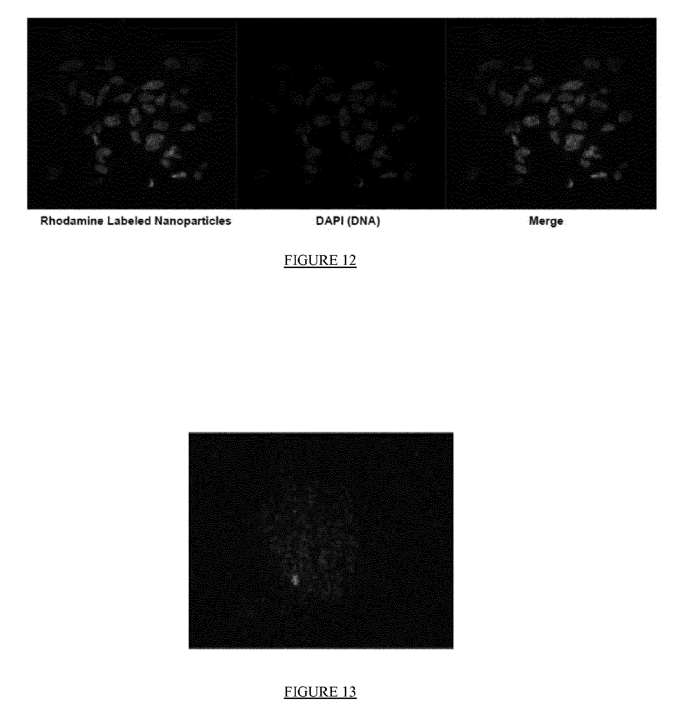

Figure 12 shows a picture of undifferentiated glioblastoma multiforme derived cancer stem cells that were treated with a nanoparticle coupled to DBA lectin, which recognizes GalNAc. The nanoparticle was incubated with the cells for 24 hours and was internalized and dissolved and diffused within the cell and appear as a reddish color (Rhodamine). The nuclei are stained in blue (DAPI) and the final image is a merger of the two.
Figure 13 shows a picture of undifferentiated glioblastoma multiforme derived cancer stem cells treated with a nanoparticle coupled to GSL II lectin, which recognizes GlcNAc. After 12 hour of exposure the nanoparticle can be seen in a slightly dissolved state appearing to be reddish. The cell nucleus appears blue (DAPI).
Figure 14 illustrates an embodiment of the disclosure. Lectins in combination with an anticancer agent such as an EGFR antibody are conjugated to polymer that covers an iron oxide nanoparticle (IONP). In some embodiments, the anticancer agent is conjugated to the particle through a biodegradable linker.

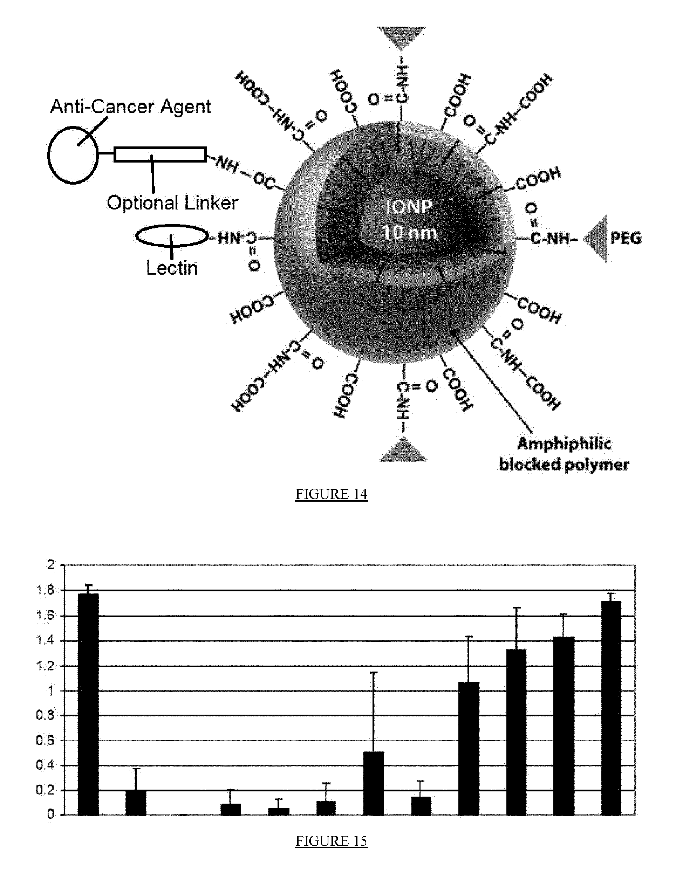

Figure 15 shows absorbance data in a MTT assay with IONPs-Cetuximab-lectin conjugates on U87wtEGFR glioblastoma cells. The lectins DOLICHOS BIFLORUS AGGLUTININ (DBA) and GRIFFONIA SIMLICIFOLIA II (GSLII) in combination with Cetuximab are conjugated to iron oxide nanoparticles. Row 1, all the way left is a control, in Row 2 IONPs-Cetuximab (0.2 mg/ml) was added, in Row 3 is IONPs-Cetuximab-DBA (0.2 mg/ml), in Row 4 IONPs-Cetuximab-GSLII (0.2 mg/ml), in Row 5 IONPs-DBA (0.2 mg/ml), in Row 6 IONPs-GSLII (0.2 mg/ml), in Row 7 IONPs-IgG-DBA (0.2 mg/ml), in Row 8 IONPs-IgG-GSLII (0.2 mg/ml), in Row 9 IONPs-IgG (0.2 mg/ml), in Row 10 Cetuximab, in Row 11 DBA, and in Row 12 GSLII.
Figure 16 shows photomicrograph of an H&E and Ki67 stained high grade glioma derived from CTB-1/DBA + GBM Neurospheres implanted in flank ofNODSCID mice. (A) solid growth pattern of high-grade glioma with high cell density and perivascular growth pattern. Ki67 labeling index was very high (greater than 40%). (B) High grade malignancy showed features of a glioblastoma including nuclear anaplasia, invasiveness and proliferation of blood vessels.

### DETAILED DISCUSSION

### Terms

As used herein, the term "combination with" when used to describe administration with an additional treatment means that the agent may be administered prior to, together with, or after the additional treatment, or a combination thereof.

As used herein, the term "derivative" refers to a structurally similar compound that retains sufficient functional attributes of the identified analogue. The derivative may be structurally similar because it is lacking one or more atoms, substituted, a salt, in different hydration/oxidation states, or because one or more atoms within the molecule are switched, such as, but not limited to, replacing a oxygen atom with a sulfur atom or replacing a amino group with a hydroxyl group. The derivative may be a prodrug. Derivatives may be prepare by any variety of synthetic methods or appropriate adaptations presented in synthetic or organic chemistry text books, such as those provide in March's Advanced Organic Chemistry: Reactions, Mechanisms, and Structure, Wiley, 6th Edition (2007) Michael B. Smith or Domino Reactions in Organic Synthesis, Wiley (2006) Lutz F. Tietze hereby incorporated by reference.

As used herein, the terms "treat" and "treating" are not limited to the case where the subject (e.g. patient) is cured and the disease is eradicated. Rather, embodiments of the present disclosure also contemplate treatment that merely reduces symptoms, and/or delays disease progression.

As used herein, "subject" refers to any animal, preferably a human patient, livestock, or domestic pet.

The terms "protein" and "polypeptide" refer to compounds comprising amino acids joined via peptide bonds and are used interchangeably.

As used herein, where "amino acid sequence" is recited herein to refer to an amino acid sequence of a protein molecule. An "amino acid sequence" can be deduced from the nucleic acid sequence encoding the protein. However, terms such as "polypeptide" or "protein" are not meant to limit the amino acid sequence to the deduced amino acid sequence, but include post-translational modifications of the deduced amino acid sequences, such as amino acid deletions, additions, and modifications such as glycolsylations and addition of lipid moieties.

The term "portion" when used in reference to a protein (as in "a portion of a given protein") refers to fragments of that protein. The fragments may range in size from four amino acid residues to the entire amino sequence minus one amino acid.

The term "chimera" when used in reference to a polypeptide refers to the expression product of two or more coding sequences obtained from different genes, that have been cloned together and that, after translation, act as a single polypeptide sequence. Chimeric polypeptides are also referred to as "hybrid" polypeptides. The coding sequences includes those obtained from the same or from different species of organisms.

The term "fusion" when used in reference to a polypeptide refers to a chimeric protein containing a protein of interest joined to an exogenous protein fragment (the fusion partner). The fusion partner may serve various functions, including enhancement of solubility of the polypeptide of interest, as well as providing an "affinity tag" to allow purification of the recombinant fusion polypeptide from a host cell or from a supernatant or from both. If desired, the fusion partner may be removed from the protein of interest after or during purification.

The terms "variant" when used in reference to a polypeptide refer to an amino acid sequence that differs by one or more amino acids from another, usually related polypeptide. The variant may have "conservative" changes, wherein a substituted amino acid has similar structural or chemical properties. One type of conservative amino acid substitutions refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagineglutamine. A variant may have "non-conservative" changes (e.g., replacement of a glycine with a tryptophan). Similar minor variations may also include amino acid deletions or insertions (in other words, additions), or both, e.g., chimera or fusion. Guidance in determining which and how many amino acid residues may be substituted, inserted or deleted without abolishing biological activity may be found using computer programs well known in the art, for example, DNAStar software. Variants can be tested in functional assays. Preferred variants have less than 10%, and preferably less than 5%, and still more preferably less than 2% changes (whether substitutions, deletions, and so on).

The term "purified" refers to molecules e.g., amino acid sequences, that are removed from their natural environment, isolated or separated. "Substantially purified" molecules are at least 60% free, preferably at least 75% free, and more preferably at least 90% free from other components with which they are naturally associated. As used herein, the term "purified" or "to purify" also refers to the removal of contaminants from a sample. The removal of contaminating proteins results in an increase in the percent of polypeptide of interest in the sample. In another example, recombinant polypeptides are expressed in plant, bacterial, yeast, or mammalian host cells and the polypeptides are purified by the removal of host cell proteins; the percent of recombinant polypeptides is thereby increased in the sample.

The term "sample" is used in its broadest sense. In one sense it can refer to a plant cell or tissue. The term "sample" is used in its broadest sense. In one sense it can refer to a biopolymeric material. In another sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like.

### Saccharide Binding Moieties

Naturally occurring lectins are sugar binding proteins that play a role in biological recognition. However, a variety of proteins, molecules, and conjugates are capable of preferentially binding a sugar or sugars are contemplated saccharide binding moieties. Saccharide binding proteins include lectins, antibodies, antibody fragments, antibody chimera, antibody mimetics, and aptamers. Non-protein based saccharide binding moieties are also contemplated such as boronic acid containing moieties, such as boronic acid containing bicyclic carbocycles, aromatics, and heterocycles. Certain crown ethers, polyazamacrocycles, cyclodextrins, tripodal polypyridines, and polyhydroxylated steroids are known saccharide binders. See, e.g., Meng et al., Chem Biol Drug Des, 2011, 78:816-825, James et al., Boronic Acids in Saccharide Recognition, 2006, Royal Society of Chemistry, and Schrader & Hamilton, Functional Synthetic Receptors, 2006, John Wiley & Sons, Chapter 2 entitled Carbohydrate Receptors, all hereby incorporated by reference in their entirety.

In certain embodiments, the disclosure contemplates saccharide binding moieties or proteins in any of the disclosed embodiments that are antibodies or fragments or chimera, antibody mimetics, or aptamers or any molecular entity that selectively binds saccharides that are more prevalent on cancer cells.

Numerous methods known to those skilled in the art are available for obtaining antibodies or antigen-binding fragments thereof. For example, antibodies can be produced using recombinant DNA methods (U.S. Patent No. 4,816,567). Monoclonal antibodies may also be produced by generation of hybridomas in accordance with known methods. Hybridomas formed in this manner are then screened using standard methods, such as enzyme-linked immunosorbent assay (ELISA) and surface plasmon resonance analysis, to identify one or more hybridomas that produce an antibody that specifically binds with a specified antigen. Any form of the specified antigen may be used as the inmunogen, e.g., recombinant antigen, naturally occurring forms, any variants or fragments thereof, as well as antigenic peptide thereof.

The modular structure of antibodies makes it possible to remove constant domains in order to reduce size and still retain antigen binding specificity. Engineered antibody fragments allow one to create antibody libraries. A single-chain antibody (scFv) is an antibody fragment where the variable domains of the heavy (V_{H}) and light chains (V_{L}) are combined with a flexible polypeptide linker. The scFv and Fab fragments are both monovalent binders but they can be engineered into multivalent binders to gain avidity effects. One exemplary method of making antibodies and fragments includes screening protein expression libraries, e.g., phage or ribosome display libraries. Phage display is described, for example, in U.S. Patent No. 5,223,409.

In addition to the use of display libraries, the specified antigen can be used to immunize a non-human animal, e.g., a rodent, e.g., a mouse, hamster, or rat. In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. U.S. Patent No. 7,064,244.

Humanized antibodies may also be produced, for example, using transgenic mice that express human heavy and light chain genes, but are incapable of expressing the endogenous mouse immunoglobulin heavy and light chain genes. Winter describes an exemplary CDR-grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by U.S. Patent No. 5,585,089; U.S. Patent No. 5,693,761; U.S. Patent No. 5,693,762; U.S. Patent No. 5,859,205; and U.S. Patent No. 6,407,213. Those methods include isolating, manipulating, and expressing the nucleic acid sequences that encode all or part of immunoglobulin Fv variable domains from at least one of a heavy or light chain. Such nucleic acids may be obtained from a hybridoma producing an antibody against a predetermined target, as described above, as well as from other sources. The recombinant DNA encoding the humanized antibody molecule can then be cloned into an appropriate expression vector.

In certain embodiments, a humanized antibody is optimized by the introduction of conservative substitutions, consensus sequence substitutions, germline substitutions and/or backmutations. An antibody or fragment thereof may also be modified by specific deletion of human T cell epitopes or "deimmunization" by the methods disclosed in U.S. Patent No. 7,125,689 and U.S. Patent No. 7,264,806. Briefly, the heavy and light chain variable domains of an antibody can be analyzed for peptides that bind to MHC Class II; these peptides represent potential T-cell epitopes. For detection of potential T-cell epitopes, a computer modeling approach termed "peptide threading" can be applied, and in addition a database of human MHC class II binding peptides can be searched for motifs present in the VH and VL sequences. These motifs bind to any of the 18 major MHC class II DR allotypes, and thus constitute potential T cell epitopes. Potential T-cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. The V BASE directory provides a comprehensive directory of human immunoglobulin variable region sequences. These sequences can be used as a source of human sequence, e.g., for framework regions and CDRs. Consensus human framework regions can also be used, e.g., as described in U.S. Patent No. 6,300,064.

Antibody mimetics or engineered affinity proteins are polypeptide based saccharide binding proteins that can specifically bind to saccharides but are not specifically derived from antibody V_{H} and V_{L} sequences. Typically, a protein motif is recognized to be conserved among a number of proteins. One can artificially create libraries of these polypeptides with amino acid diversity and screen them for binding to saccharides through phage, yeast, bacterial display systems, cell-free selections, and non-display systems. See Gronwall & Stahl, J Biotechnology, 2009, 140(3-4), 254-269, hereby incorporated by reference in its entirety. Antibody mimetics include affibody molecules, affilins, affitins, anticalins, avimers, darpins, fynomers, kunitz domain peptides, and monobodies.

Affibody molecules are based on a protein domain derived from staphylococcal protein A (SPA). SPA protein domain denoted Z consists of three α-helices forming a bundle structure and binds the Fc protion of human IgG1. A combinatorial library may be created by varying surface exposed residues involved in the native interaction with Fc. Affinity proteins can be isolated from the library by phage display selection technology. See Orlova et al., Cancer Res., 2007, 67:2178-2186, hereby incorporated by reference in its entirety.

Monobodies, sometimes referred to as adnectins, are antibody mimics based on the scaffold of the fibronectin type III domain (FN3). See Koide et al., Methods Mol. Biol. 2007, 352: 95-109, hereby incorporated by reference in its entirety. FN3 is a 10 kDa, β-sheet domain, that resembles the V_{H} domain of an antibody with three distinct CDR-like loops, but lack disulfide bonds. FN3 libraries with randomized loops have successfully generated binders via phage display (M13 gene 3, gene 8; T7), mRNA display, yeast display and yeast two-hybrid systems. See Bloom & Calabro, Drug Discovery Today, 2009, 14(19-20):949-955, hereby incorporated by reference in its entirety.

Anticalins, sometimes referred to as lipocalins, are a group of proteins characterized by a structurally conserved rigid β-barrel structure and four flexible loops. The variable loop structures form an entry to a ligand-binding cavity. Several libraries have been constructed based on natural human lipocalins, i.e., ApoD, NGAL, and Tlc. See Skerra, FEBS J., 275 (2008), pp. 2677-2683, hereby incorporated by reference in its entirety.

The ankyrin repeat (AR) protein is composed repeat domains consisting of a β-turn followed by two α-helices. Natural ankyrin repeat proteins normally consist of four to six repeats. The ankyrin repeats form a basis for darpins (designed ankyrin repeat protein) which is a scaffold comprised of repeats of an artificial consensus ankyrin repeat domain. Combinatorial libraries have been created by randomizing residues in one repeat domain. Different numbers of the generated repeat modules can be connected together and flanked on each side by a capping repeat. The darpin libraries are typically denoted NxC, where N stands for the N-terminal capping unit, C stands for the C-tenninal capping domain and x for the number of library repeat domains, typically between two to four. See Zahnd et al., J. Mol. Biol., 2007, 369:1015-1028, hereby incorporated by reference in its entirety.

Aptamers refer to affinity binding molecules identified from random proteins or nucleic acid libraries. Peptide aptamers have been selected from random loop libraries displayed on TrxA. See Borghouts et al., Expert Opin. Biol. Ther., 2005, 5:783-797, hereby incorporated by reference in its entirety. SELEX ("Systematic Evolution of Ligands by Exponential Enrichment") is a combinatorial chemistry technique for producing oligonucleotides of either single-stranded DNA or RNA that specifically bind to a target. Standard details on generating nucleic acid aptamers can be found in U.S. Patent No. 5,475,096, and U.S. Patent No. 5,270,163. The SELEX process provides a class of products which are referred to as nucleic acid ligands or aptamers, which has the property of binding specifically to a desired target compound or molecule. Each SELEX-identified nucleic acid ligand is a specific ligand of a given target compound or molecule. The SELEX process is based on the fact that nucleic acids have sufficient capacity for forming a variety of two- and three-dimensional structures and sufficient chemical versatility available within their monomers to act as ligands (form specific binding pairs) with virtually any chemical compound, whether monomeric or polymeric. Saccharides can serve as targets.

### Lectins Identify Glycan Biomarkers on Glioblastoma Multiforme Derived Cancer Stem Cells

A panel of lectins was used to identify differences in glycan expression found on undifferentiated and differentiated glioma derived cancer stem cells. Fluorescently labeled lectins which recognized N-acetyl galactosamine (GalNAc) and N-acetyl glucosamine (GlcNAc) were shown differentially recognize the surface of the cells, based on their state of differentiation. GalNAc and GlcNAc were shown to be highly expressed on the surface of undifferentiated cells and markedly decreased expression over a 12 day duration of differentiation. Additionally, these glycans were used as tools for sorting cancer stem cell populations from populations positive for differentiation. These results suggest that GalNAc and GlcNAc are novel biomarkers for identifying glioma derived cancer stem cells and can be used to isolate cancer stem cells from unsorted cell population.

Previous studies have shown a higher re-occurrence rate of the same tumor after treatment. Several studies have acknowledged the presence of stem-like cells in brain tumor tissue cultures, which are CD133 positive and have the ability to give rise to all cell types found within the tumor, potentially perpetuating its growth. The existence of a population of precursor cells or cancer stem cells (CSCs), which are resistant to treatment, may promote the re-occurrence of the cancerous legion. It has been discovered that a number of lectins bind undifferentiated GBM tumor derived stem cells. Lectins were used to determine the cell surface glycan expression patterns of CD133⁺ CSCs derived from a human GBM brain tumor via FACS analysis and the CD133- sorted population was used as a control. Five lectins, which recognize GalNAc, and two lectins, which recognize α-N-acetylglucosamine (GlcNAc) were highly reactive towards undifferentiated CD133⁺ GBM tumor derived cells. Presentation of GalNAc and GlcNAc on the cell surface is rapidly diminished during differentiation, in conjunction with GBM-CSC marker CD133, and general stem cell markers (OCT-4 and SOX 2). These findings establish the use of lectins and cell surface glycans (GalNAc and GlcNAc) as nondestructive markers to characterize CSCs derived from GBM brain tumors independently of CD133.

The cell surface glycan expression patterns of the human GBM brain tumor derived CD133⁺ CSC line and negative control cell line (CD133⁻) were first explored by flow cytometry. Afterwards, the lectin DBA was used to separate the two cell lines, based on the recognition of specific cell surface glycosylation, through FACS and magnetic bead separation. This study demonstrates that glycans can be used to separate GBM-derived CSCs from differentiated tumor cells in the presence or absence of the previously defined biomarker CD133⁺.

The resolution of lectins as markers is emphasized by their ability to discriminate between GBM-derived CSCs and differentiated tumor cells. Because differentiated tumor cells have lost their ability to self-renew, lectins are likely to have utility as an indicator of differentiation commitment. The current GBM stem cell marker CD133 cannot be used for this. Interestingly, the only glycans to change substantially on the cell surface during differentiation were GalNAc and GlcNAc. It will be interesting to determine how GalNAc and GlcNAc epitopes are regulated on the surface of GBM-derived CSCs as they differentiate and to determine the identity of proteins and lipids marked with the glycans. The current marker for GBM-derived CSCs, CD133, is highly expressed and upon differentiation it rapidly decreases expression. However, CD133 expression is greatly influenced by various environmental and genetic factors. GBM-derived cells which were negative for CD133 expression have been shown to promote tumor formation. Because GalNAC and GlcNAc epitopes are downregulated concertedly or slightly before CD133, one can define cell populations with greater temporal resolution. In combination with CD133, lectins can be used to define in detail the early stages of differentiation in GBM-derived CSC populations.

GalNAc and GlcNAc are nondectructive markers for GBM derived CSCs cells and are a reliable readout for initial differentiation events, at a level of temporal resolution that was not previously possible. Lectins can be used as recognition tools for these glycan markers which are located on the surface of cells and therefore allowing for cell sorting, culturing and harvesting a specific cell population. This offers an alternative to previous antibody methodologies which are limiting because they require destructive techniques. These markers can also be used for sorting cancer stem cells as well.

### Drug Targeting

Targeting drugs to a specific site provides several advantages over non-specific sites such as preventing side effects of drugs while enhancing uptake by targeted cells. As a result of their specificity, there is a natural basis for the use of lectin-glycan pairs in drug targeting. As mentioned above, GalNAc and GlcNAc are expressed on GBM-derived CSCs. A reverse lectin targeting approach can utilize exogenous lectins as targeting moieties that steer drugs and their delivery system to specific glycans that are expressed on GBM-derived CSC. Lectin targeting was carbohydrate specific with high binding affinity and internalization of cell surface-bound lectin was confirmed. This may show equal promise for the development of functionalized drug delivery systems for site specific therapy targeting GBM-derived CSCs. This approach may prevent tumor reoccurrences which are believe to be associated with the presence of CSCs.

Within certain embodiments it is contemplated that saccharide binding proteins disclosed herein may be conjugated to polymers comprising ethylene glycol monomer and lactone monomers. Xin et al., International Journal of Pharmaceutics, 2010, 402, 238-247, hereby incorporated by reference, disclose an anti-tumor effect of paclitaxel (PTX)-loaded methoxy poly(ethylene glycol)-poly(ε-caprolactone) nanoparticles (MPEG-NP/PTX) against glioblastoma multiforme (GBM).

Within certain embodiments it is contemplated that saccharide binding proteins disclosed herein may be conjugated to polymers coating a metal nanoparticle. In one example, quantum dots may be prepared with amphiphilic multidentate ligands and modified with saccharide binding proteins. See Kairdolf et al., J Am Chem Soc, 2008, 130, 12866-12867 and US Published Patent Application Number 2011/0260111. Pegylated colloidal gold and iron oxide nanoparticles are also contemplated. See Kairdolf & Nie, J Am Chem Soc, 2011, 133(19):7268-7271, Qian et al., Nature Biotechnology, 2008, 26, 83 - 90, Hadjipanayis et al., Cancer Research, 2010, 70(15):6303-6312, and Peng et al., Int J Nanomedicine, 2008, 3(3): 311-321, all hereby incorporated by reference in their entirety.

Contemplated saccharide binding proteins include Dolichos biflorus lectin chain A (SEQ ID NO:2) ADIQSFSFKN FNSSSFILQG DATVSSSKLR LTKVKGNGLP TLSSLGRAFY SSPIQIYDKS 61 TGAVASWATS FTANIFAPNK SSSADGIAFA LVPVGSEPKS NSGFLGVFDS DVYIDNSAQTV 121 AVEFDTFSNT DWDPTSRHIG IDVNSIKSIR TASWGLANGQ NAEILITYNA ATSLLVASLV 181 HPSRRTSYIV SERVDITNEL PEYVSIGFSA TTGLSEGYTE THDVLSWSFA SKLPDDSTTE 241 PLDIASYLVR NVL or variants, e.g., conserved variants, or portions thereof that bind α-linked N-acetylgalactosamine.

Contemplated saccharide binding proteins include peanut lectin (SEQ ID NO:3) MKPFCVFLTF FLLLAASSKK VDSAETVSFN FNSFSEGNPA INFQGDVTVL SNGNIQLTNL 61 NKVNSVGRVL YAMPVRIWSS ATGNVASFLT SFSFEMKDIK DYDPADGIIF FIAPEDTQIP 121 AGSIGGGTLG VSDTKGAGHF VGVEFDTYSN SEYNDPPTDH VGIDVNSVDS VKTVPWNSVS 181 GAVVKVTVIY DSSTKTLSVA VTNDNGDITT IAQVVDLKAK LPERVKFGFS ASGSLGGRQI 241 HLIRSWSFTS TLITTTRRSI DNNEKKIIVINM ASA, or variants, e.g., conserved variants, or portions thereof that bind galactosyl (β-1,3) N-acetylgalactosamine.

Contemplated saccharide binding proteins include soybean lectin (SEQ ID NO:4) AETVSFSWNK FVPKQPNMIL QGDAIVTSSG KLQLNKVDEN GTPKPSSLGR ALYSTPIHIW 61 DKETGSVASF AASFNFTFYA PDTKRLADGL AFFLAPIDTK PQTHAGYLGL FNENESGDQV 121 VAVEFDTFRN SWDPPNPHIG INVNSIRSIK TTSWDLANNK VAKVLITYDA STSLLVASLV 181 YPSQRTSNIL SDVVDLKTSL PEWVRIGFSA ATGLDIPGES HDVLSWSFAS NLPHASSNID 241 PLDLTSFVLH EAI or variants, e.g., conserved variants, or portions thereof that bind terminal α- or β-linked N-acetylgalactosamine.

Contemplated saccharide binding proteins include isolectin I-A of Griffonia (Bandeiraea) simplicifolia (SEQ ID NO:5) FNLPNFWSDV KDNIIFQGDA NTTAGTLQLC KTNQYGNPLQ YRAGRALYSD PVQLWDNKTG 61 SVASFYTEFT FFLKITGDGP ADGLAFFLAP PDSDVKDAGA YLGLFNKSTA TQPSKNQVVA 121 VEFDTWKNTD FPEPSYRHIG INVNSIVSVA TKRWEDSDIF SGKIATARIS YDGSAKILTV 181 VLSYPDGADY ILSHSVDLSK NLPNPIRVGI SASTGANQFL TVYVLSWRFS SALQSTSVNA 241 AMGPEIIRTV V or variants, e.g., conserved variants, or portions thereof that bind alpha GalNAc.

Contemplated saccharide binding proteins include Griffonia simplicifolia lectin isoform II (SEQ ID NO:6) MAKSTAKPNF SLLLPILISL FLFQLNRVKS ADTVCFTSTS FGKDVSDLTLQ or variants, e.g., conserved variants, or portions thereof that bind N-acetylglucosamine.

Contemplated saccharide binding proteins include Erythrina cristagalli lectin (SEQ ID NO: 7) VETISFSFSE FEPGNNDLTL QGAAIITQSG VLQLTKINQN GMPAWDSTGR TLYTKPVHIW 61 DMTTGTVASF ETRFSFSIEQ PYTRPLPADG LVFFMGPTKS KPAQGYGYLG VFNNSKQDNS 121 YQTLAVEFDT FSNPWDPPQV PHIGIDVNSI RSIKTQPFQL DNGQVANVVIKYDASSKILL 181 AVLVYPSSGA IYTIAEIVDV KQVLPEWVDV GLSGATGAQR DAAETHDVYS WSFHASLPET 241 ND or variants, e.g., conserved variants, or fragments that bind galactosyl (β-1,4) N-acetylglucosamine.

The polymer may also be covalently attached to polypeptides that provide access to crossing the blood brain barrier. For example, Xin et al., Biomaterials, 2011, 1-13, hereby incorporated by reference, have also disclosed peptide-conjugated poly(ethylene glycol)-co-poly(e-caprolactone) nanoparticles act as a targeting drug delivery system for brain glioma. It is disclosed that angiopep protein (SEQ ID NO:1)TFFYGGSRGKRNNFKTEEY, improves the ability for the polymers to cross the blood-brain barrier.

### EXPERIMENTAL

### Example 1: Cell Sorting Of CD133⁺ GBM-derived CSCs Using Magnetic Bead Separation

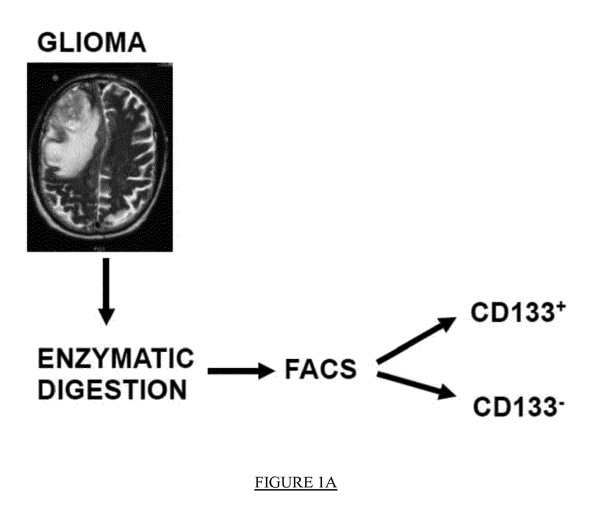

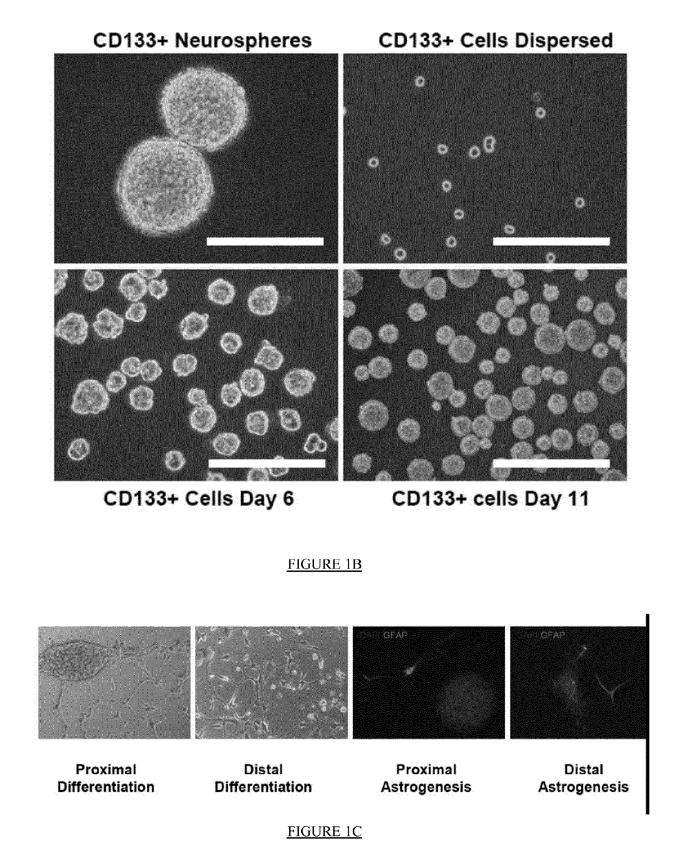

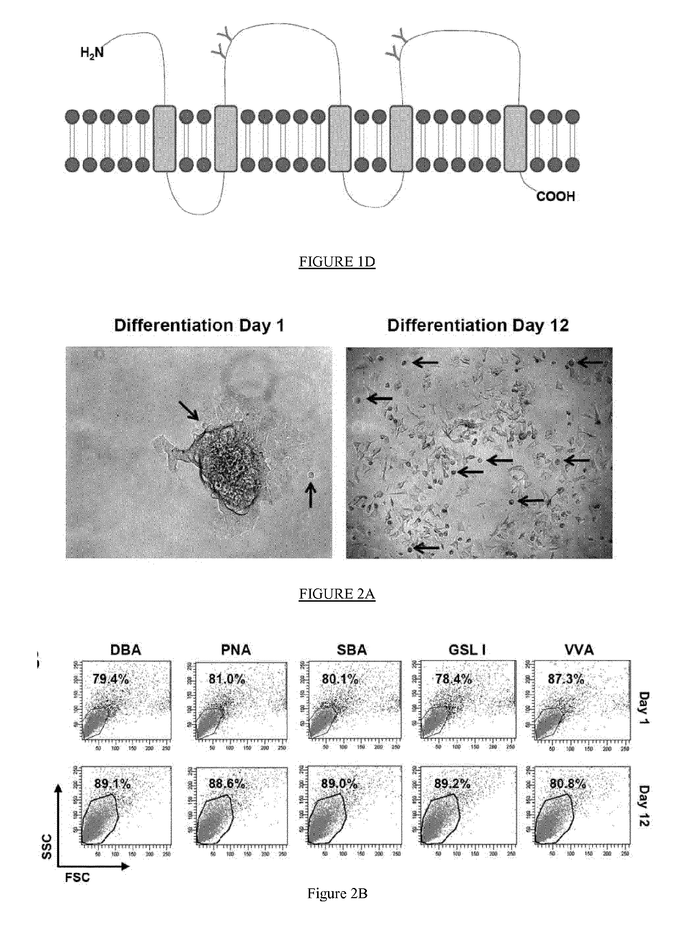

Enzymatically digested tumor, while in single cell suspension was labeled with biotinylated CD133 antibody. Metallic beads coupled with strepavidin were added to the mixture and cell which were positive for CD133 were successfully separated from the total population. It has been reported that CD133⁺ GBM-derived CSCs make up approximately 0.1%-10% of the tumor, however the average is <1%. After multiple sorts, our CD133⁺ positive population averaged 40-50% of the total population collected, which was less than 1% of the total tumor. The CD133⁺ cells formed neurospheres and were grown until they reached 150µM in diameter, at which they can be passaged or frozen and stored. Afterwards, the neurospheres were plated onto chamber slides where they successfully differentiated into TUJ-1 positive neuronal lineages as well as glial cells (Astrocytes) (Figure 1). This result shows that we are able to isolate CD133⁺ GBM-derived CSCs from our tumor and these isolated cells are capable of differentiating in neuronal and glial cell lineages.

### Example 2: Lectins Identify GalNAc and GIcNAc on the surface of CD133⁺ GBM-CSCs

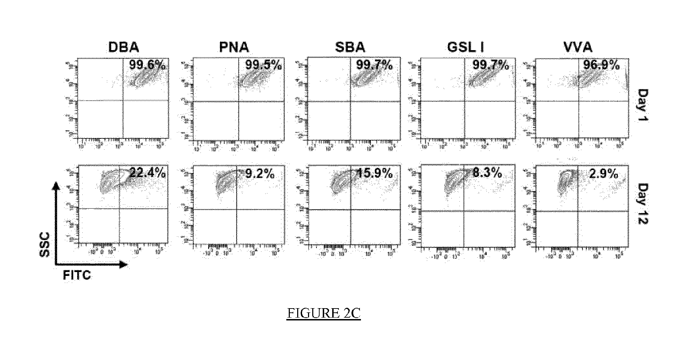

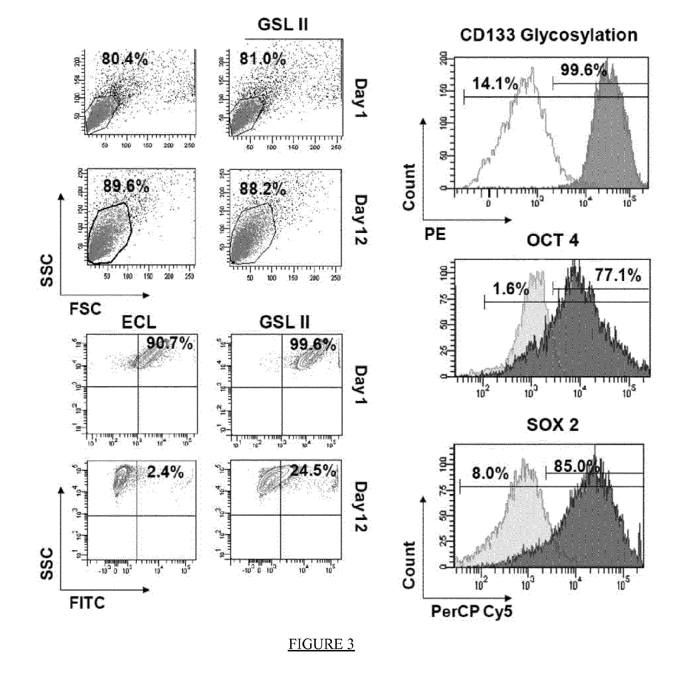

The fact that lectin moieties recognize carbohydrates located on the surface of cells makes them a great tool to identify changes in glycosylation patterning during differentiation. We used a panel of 20 lectins to study the patterning of CD133⁺ GBM-CSCs surface glycosylation during differentiation (Table 1). Neurospheres derived from CD133⁺ GBM-CSCs were grown as mentioned above and were plated onto polyornithin and laminin coated tissue culture grade plastics. Within 10 hours the neuroshperes adhered and neurite formations were observed. Differentiation continued for 12 days, and harvested cells (day 1 and day 12) were labeled with our panel of lectins (Figure 2A). A quantitative evaluation of the cell populations show lectins DBA, PNA, SBA, GSL I, and VVA, all of which recognize the glycan moiety GalNAc, highly recognized undifferentiated GBM-CSCs. However, this recognition markedly decreased after 12 days of differentiation (Figures 2A and 2B). Similarly lectins ECL and GSL II, which recognize the glycan moiety GlcNAc, highly recognized undifferentiated GBM-CSCs. This recognition noticeably decreased after 12 days of differentiation (Figure 3A and 3B). Furthermore, the recognition and decrease in recognition of GalNAc and GlcNAc coincide with the expression of GBM-CSC marker CD133-glycosylation (CD133-G) and general stem cell markers OCT 4 and SOX2 (Figure 3C). OCT 4 and SOX2 have been well studied in GBMs and are accepted indicators of stemness in GBM cell lines. This result shows that specific lectins can identify glycans GalNAc and GlcNAc which are uniquely expressed on the surface of GBM-CSCs. This result is derived through non-destructive techniques and correlate to the invasive (destructive) analysis of general stem cell markers OCT 4 and SOX 2.

### Example 3: Fluorescently labeled DBA and Cell Surface Glycan GalNAc Can Be Used To Sort GBM-CSCs To Establish New Cell Lines and Have Less Proliferation but Greater Apoptosis

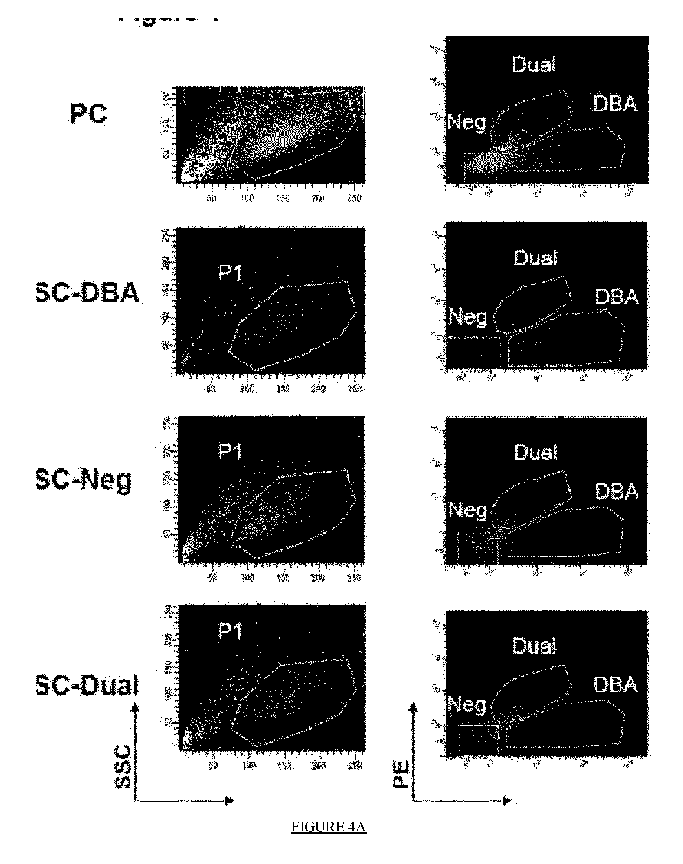

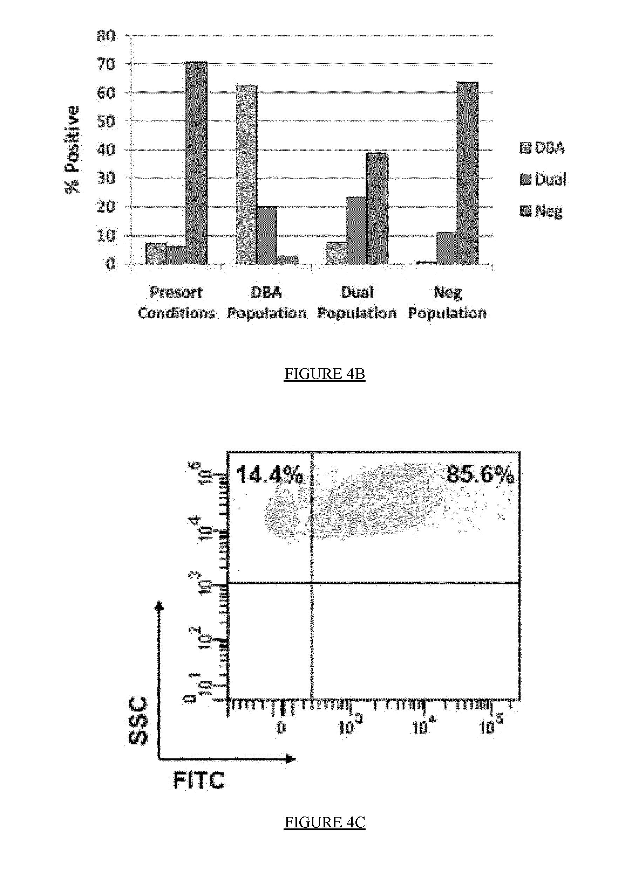

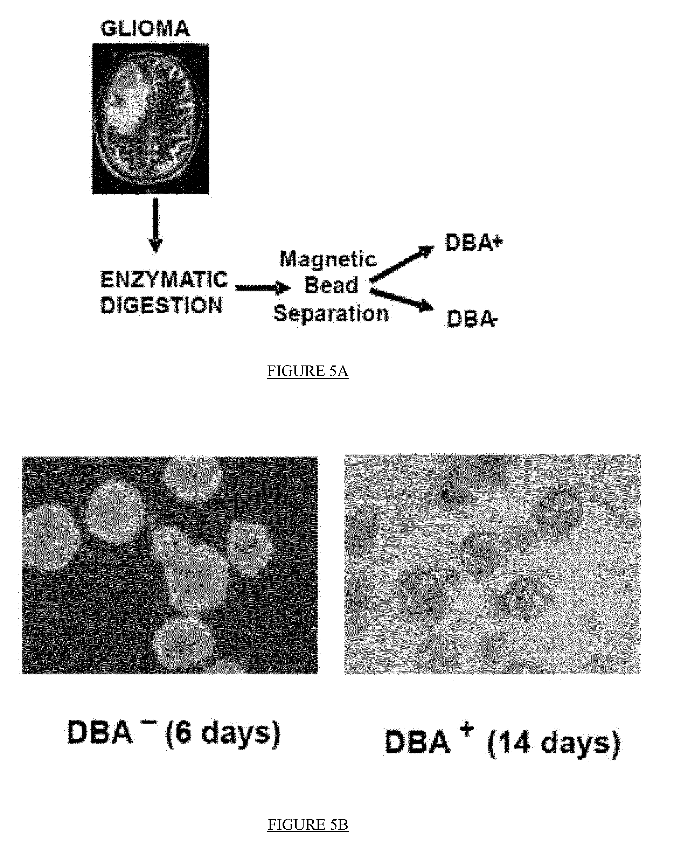

GBM cells, in single cell suspension, were treated with fluorescently labeled DBA lectin and two individual technologies were employed to separate positively labeled cells from the parent population. BD FACS Aria cell sorter successfully separated and quantitatively analyzed positively labeled cells for DBA from non-labeled (Neg. Population) and Dual labeled cells (Fluorescently labeled DBA and fluorescently labeled actin antibody) (Figure 4A and 4B). DBA has specificity for GalNAc and can be competed off of cells using 200mM free (unbound) GalNAc (Figure 4C), suggesting that non-specific interaction between DBA lectin and proteins or random glycans is very unlikely. Contamination (bacterial) being of great concern, a second, more sterile, method was developed using the autoMACS Pro Separator (Miltenyi Biotec). This benchtop automated magnetic cell sorter was used in a sterile tissue culture cabinet and has a proven record for the isolation of virtually any cell type from any species. Using auto MACS magnetic beads which recognized fluorescent labeling one is able to separate DBA positive labeled cells from non-labeled. See Figure 5A.

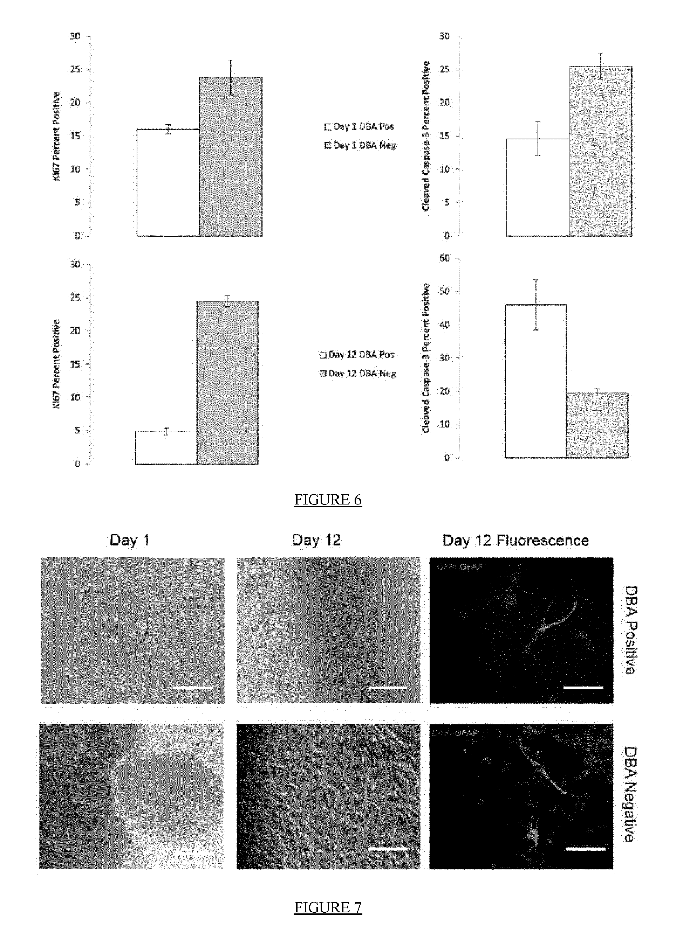

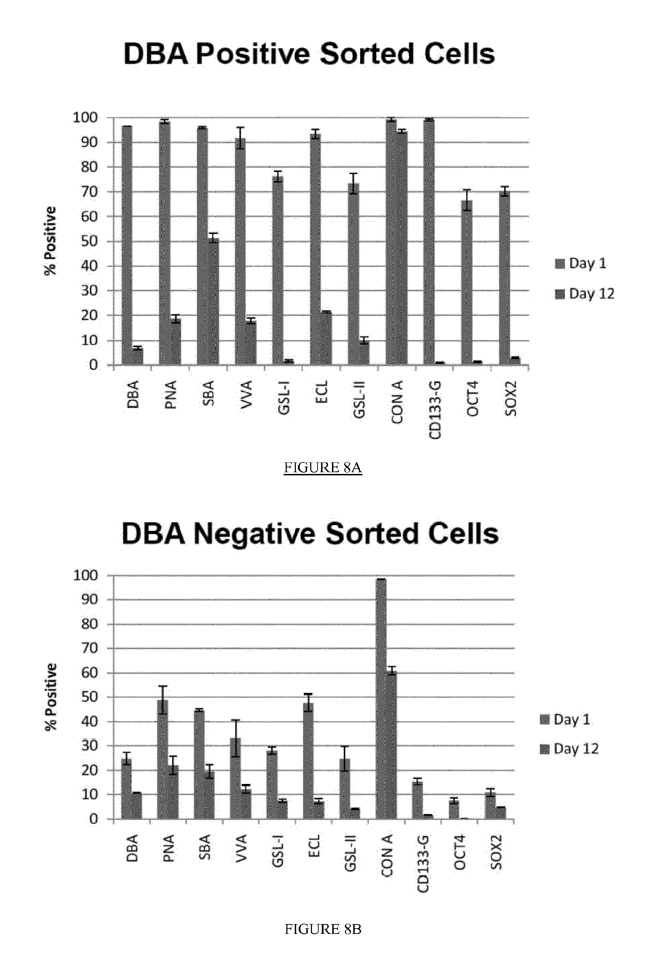

DBA lectin positive cells made up less than 1% of the total population and phenotypically appeared to proliferate at a much slower rate than the DBA negative population. See Figure 5B. Measuring the diameter of the neurospheres confirmed that at greater than twice the time course, cells positively sorted for DBA were smaller suggesting slower proliferation or increased cell death (Figure 5C). Ki67 positive staining is a hallmark of proliferation and day 1 DBA negative populations showed higher Ki67 antibody reactivity (23.8%) versus the DBA positive population (16%) (Figure 6). This trend continued throughout differentiation and by day 12, 24.5% of DBA negative sorted cells were Ki67 positive, meanwhile DBA positive sorted cells showed a reduction in reactivity to KI67 (4.9%). Cleaved caspase 3, an indicator of apoptosis, is elevated in the DBA negative population at day 1 (25.5) versus the DBA positive population (14.6), however, by day 12 apotosis appears to increase in the DBA positive population to almost half (46%) while the DBA negative population shows a decrease in cell death (19.6%) (Figure 6). Increased proliferation in DBA negative populations can be visualized via microscopy at day 1 and day 12 (Figure 7). At day 12 the DBA negative population appears to be denser than the DBA positive population (Figure 7), however, both populations are able to produce GFAP positive cells (Astrocytes) identical to the CD133 positive sorted cells (Figure 7). Positively sorted DBA cells show high reactivity for lectins DBA, PNA, SBA, WA, GSL-I, which recognize GalNAc, and decrease by day 12 (Figure 8A). GSL-II and ECL, which recognize GlcNAc, are highly reactive as well in the DBA positive sorted cells and also decreased by day 12 (Figure 8A). DBA negative sorted cells contradict the aforementioned, and show low reaction towards GalNAc and GlcNAc recognizing lectins and this continues to decrease by day 12 (Figure 8B). Con A remains high throughout in both populations and serves as a positive control (Figures 8A and 8B). However, GBM-CSC marker CD133-G and general stem cell markers OCT 4 and SOX 2 are high in the DBA positive sorted cells, and similar to DBA reactivity, decreases substantially by day 12 of differentiation (Figure 8A). However, in the DBA negative population these stem cell markers uniformly are low at day one and are almost non-existent by day 12 (Figure 8A). Until now CD133 has been considered the only marker for CSCs derived from GBM brain tumors. However, using cell sorting technology, DBA lectin, which recognizes GalNAc, we were able to separate CD133⁺ cells from CD133- cells. Also, this data shows proliferation is higher in GBM cells that are more differentiated, however during differentiation GBM-CSC become more apoptotic. This data shows that lectins that recognize GalNAc can be used to identify and sort GBM-CSCs.

### Example 4: DBA Lectin Sorted GBM-CSCs Produce high grade gliomas in NOD/SCID Mice

CD133+ (CTB/ CD133+) sorted GBM-CSCs were intracranially implanted (1x10⁵) in NOD/SCID mice to demonstrate tumorigenicity. At six months, an infiltrative high grade glioma was noted confirming the orthotopic tumorigenicity of these cells. To determine the tumorigenicity of DBA+ sorted cells (CTB-1/DBA+), 1x10⁴ cells were implanted into the flank of NOD/SCID mice.

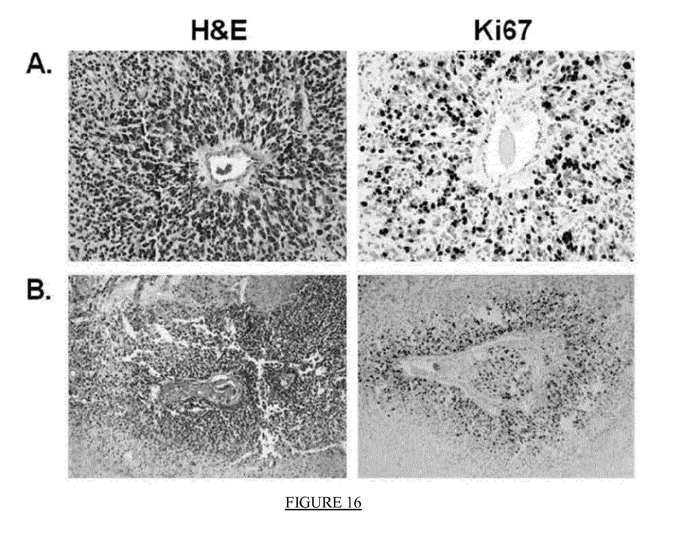

These CTB-1/DBA+ cells produced a high-grade neoplasm with features of a glioblastoma, including high cell density, an infiltrative pattern and angiogenesis (Fig 16A and B (H+E stain)). DBA+ neurosphere derived tumors also display high immune-reactivity with the Ki67 antibody confirming their high proliferation rate. See Figure 16.

### Example 5: GalNAc Is A Unique Structure and Is Not Part of the CD133

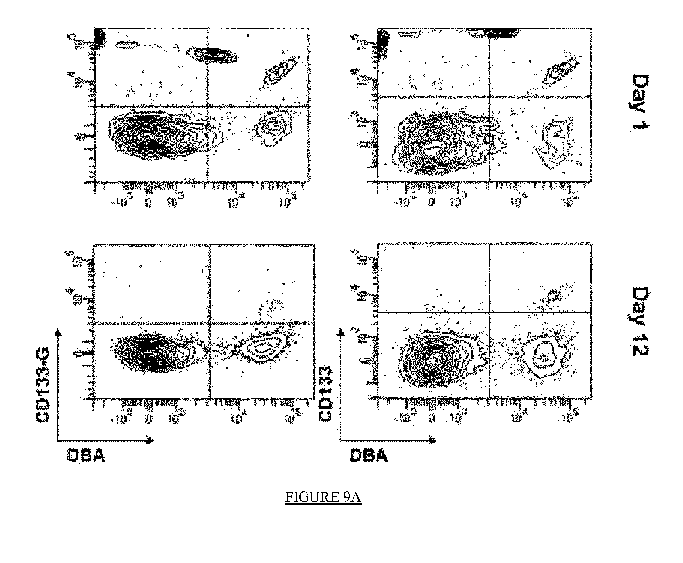

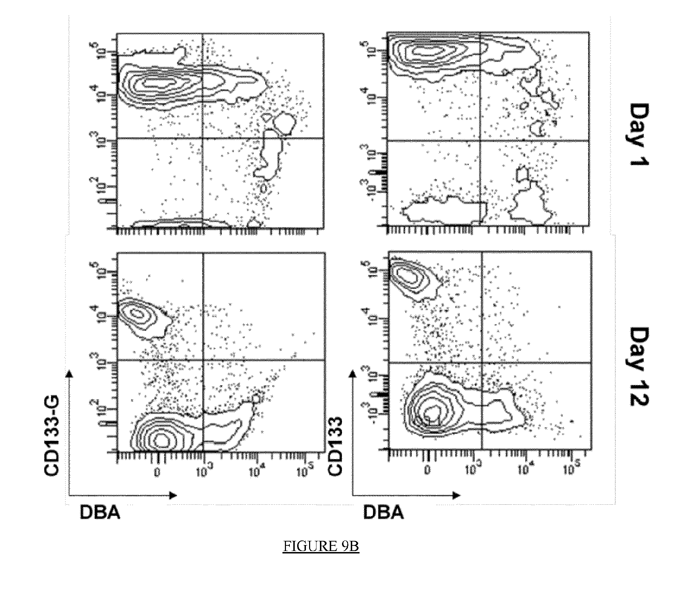

Figure 9 shows DBA sorted negative (A) and positive (B) cells and the individual populations of DBA (GalNAc) positive, CD133-G and CD133 populations. Clearly, the GalNAC positive cells are a distinct population and this marker can be used to isolate and sort stem cells.

### Example 6: Nanoparticle preparation

Nanoparticles with sizes of approximately 200 nm are formulated by first, suspending the solid block copolymer in tetrahydrofuran (THF) at 10 mg per ml of solvent. This organic phase is then added drop-wise to a vigorously stirred 0.01 M pH 6.5 PBS solution. The resulting organic in water emulsion is then allowed to stiff for 30 min to allow the particles to set-up. The emulsion is then stirred under reduced pressure via a rotary evaporator to remover remaining THF. The aqueous solution containing the now hardened particles is then added to two equivalents of a stirred 0.1 M pH 7.4 PBS solution. Lectins or BSA are then added to this solution and react to the amine reactive groups on the particles. The reaction is allowed to continue for 2 hours. Protein conjugated particles are washed of any free protein via centrifugation. The particles are then washed 2X via resuspention in PBS and subsequent centrifugation. Finally, the particles are lyophilized for long term storage.

### Example 7: Lectins conjugated in combination with Cetuximab to iron oxide nanoparticles kill U87wtEGFR glioblastoma cells

Polymer coated iron oxide nanoparticles with terminal carboxylic acid groups may be obtained from OceanNanotech. Lectins DOLICHOS BIFLORUS AGGLUTININ (DBA) or GRIFFONIA SIMLICIFOLIA II (GSLII) in combination with Cetuximab are conjugated to iron oxide nanoparticles through a polymer coat that covers an iron oxide nanoparticle (IONP). Any polypeptide containing a primary amine group may be conjugated to the IONPs using following coupling procedure or as appropriately modified. A solution of Lectin/Cetuximab is mixed with an IONP comprising carboxy terminated polymer that has been activated with an EDAC/NHS mixture.

The reduction of tetrazolium salts is a reliable way to examine cell proliferation. The yellow tetrazolium MTT (3-(4, 5-dimethylthiazolyl-2)-2,5-diphenyltetrazolium bromide) is reduced by metabolically active cells to generate reducing equivalents such as NADH and NADPH. The resulting intracellular purple formazan is quantified by spectrophotometric means. The MTT Cell Proliferation Assay measures the cell proliferation rate and conversely, when metabolic events lead to apoptosis or necrosis, the reduction in cell viability. Figure 15 shows data on lectins conjugated in combination with cetuximab, a EGFR antibody, to the polymer coat surrounding an iron oxide nanoparticle. The results suggest IONPs conjugated with cetuximab in combination with DBA are preferred.

The disclosure relates to a conjugate comprising: a saccharide binding moiety; a polymer; and a therapeutic agent; wherein the saccharide binding moiety is covalently attached to the polymer.

The saccharide binding moiety may be a lectin selected from the group consisting of concanavalin A (CON A), dolichos biflorus agglutinin (DBA), peanut agglutinin (PNA), ricinus communis agglutinin I (RCA 120), soybean agglutinin (SBA), ulex europaeus agglutinin I (UEA I), wheat germ agglutinin (WGA), griffonia simplicifolia lectin I (GSL I), lens culinaris agglutinin (LCA), phaseolus vulgaris erythroagglutinin (PHA-E), phaseolus vulgaris leucoagglutinin (PHA-L), pisum sativum agglutinin (PSA), griffonia simplicifolia lectin II (GSL II), datura stramonium lectin (DSL), erythrina cristagalli lectin (ECL), Jacalin, lycopersicon esculentum lectin (LEL), solanum tuberosum lectin (STL), and vicia villosa lectin (VVA).

The saccharide binding moiety may bind a saccharide or polysaccharide selected from the group consisting of α- or β-lined N-acetylgalactosamine, branched and terminal α-linked mannose, α-linked N-acetylgalactosamine, galactosyl (β-1,3) N-acetylgalactosamine, oligosaccharides ending in galactose, terminal α- or β-linked N-acetylgalactosamine, α-linked fucose residues, terminal N-acetylglucosamine or chitobiose, α-N-acetylgalactosamine and α-galactose residues, α-linked mannose residues, galactose, α-linked mannose residues, α- or β-linked N-acetylglucosamine, N-acetyllactosamine, galactosyl (β-1,4) N-acetylglucosamine, galactosyl (β-1,3) N-acetylgalactosamine, N-acetylglucosamine, N-acetylglucosamine and N-acetylmuramic acid.

The polymer may form a particle comprising an outer hydrophilic coat.

The polymer may comprise lactone containing monomers and ethylene glycol containing monomers.

The therapeutic agent may be an anticancer agent.

The polymer may surround the therapeutic agent.

The therapeutic agent may be conjugated to the polymer through a biodegradable bond.

The therapeutic agent may be temozolomide, bevacizumab, doxorubicin, hydroxydaunorubicin, bleomycin, dactinomycin, vinblastine, dacarbazine, mechlorethamine, cyclophosphamide, etoposide, teniposide, vincristine, prednisone, platinum agent (cisplatin, carboplatin, oxaliplatin), fluorouracil, folinic acid, carmustine, rituximab, methotrexate, procarbazine, epirubicin, irinotecan, ifosfamide, chlorambucil, lomustine, leucovorin, fludarabine, thalidomide, dexamethasone, docetaxel, anastrozole, topotecan, combretastatin, or combretastatin A-4 phosphate.

The polymer may surround a particle.

The particle may have a diameter of between about 200 and 5 nm.

The particle may be a metal particle comprising an iron oxide particle, elemental iron coated with iron oxide, gold, silver, a quantum dot, or bismuth encapsulated in a phospholipid core.

The therapeutic agent may be an EGFR antibody.

The therapeutic agent may be cetuximab.

The saccharide binding moiety may be DBA.

The disclosure also relates to a method of treating cancer comprising administering a composition comprising the conjugate as herein described to a subject diagnosed with cancer.

The subject may be diagnosed with a brain tumor.

The subject may have previously undergone surgical removal of a tumor or radiation therapy.

The subject may be administered the composition in combination with radiation therapy.

The composition may be administered orally, by injection, convection enhance delivery, or intracerebrally.

## Claims

1. A conjugate comprising:
a) a saccharide binding moiety;
b) a polymer; and
c) a therapeutic agent;
wherein the saccharide binding moiety is covalently attached to the polymer.

2. The conjugate of Claim 1, wherein the saccharide binding moiety is dolichos biflorus agglutinin (DBA) or griffonia simplicifolia lectin II (GSL II).

3. The conjugate of Claim 1 or 2, wherein the saccharide binding moiety binds a saccharide or polysaccharide selected from the group consisting of α- or β-linked N-acetylgalactosamine, branched and terminal α-linked mannose, α-linked N-acetylgalactosamine, galactosyl (β-1,3) N-acetylgalactosamine, oligosaccharides ending in galactose, terminal α- or β-linked N-acetylgalactosamine, α-linked fucose residues, terminal N-acetylglucosamine or chitobiose, α-N-acetylgalactosamine and α-galactose residues, α-linked mannose residues, galactose, α-linked mannose residues, α- or β-linked N-acetylglucosamine, N-acetyllactosamine, galactosyl (β-1,4) N-acetylglucosamine, galactosyl (β -1,3) N-acetylgalactosamine, N-acetylglucosamine, N-acetylglucosamine and N-acetylmuramic acid.

4. The conjugate of any one of Claims 1 to 3, wherein the polymer:
a) forms a particle comprising an outer hydrophilic coat; and/or
b) comprises lactone containing monomers and ethylene glycol containing monomers.

5. The conjugate of any one of the preceding Claims, wherein the therapeutic agent is an anticancer agent.

6. The conjugate of any one of the preceding Claims, wherein polymer surrounds the therapeutic agent or the therapeutic agent is conjugated to the polymer through a biodegradable bond.

7. The conjugate of any one of the preceding Claims, wherein the therapeutic agent is temozolomide, bevacizumab, doxorubicin, hydroxydaunorubicin, bleomycin, dactinomycin, vinblastine, dacarbazine, mechlorethamine, cyclophosphamide, etoposide, teniposide, vincristine, prednisone, platinum agent (cisplatin, carboplatin, oxaliplatin), fluorouracil, folinic acid, carmustine, rituximab, methotrexate, procarbazine, epirubicin, irinotecan, ifosfamide, chlorambucil, lomustine, leucovorin, fludarabine, thalidomide, dexamethasone, docetaxel, anastrozole, topotecan, combretastatin, or combretastatin A-4 phosphate.

8. A composition comprising a conjugate of any one of Claims 1 to 7, wherein the polymer surrounds a particle.

9. The composition of Claim 8, wherein the particle has a diameter of between about 200 and 5 nm.

10. The composition of Claim 8 or 9, wherein the particle is metal particle comprising an iron oxide particle, elemental iron coated with iron oxide, gold, silver, a quantum dot, or bismuth encapsulated in a phospholipid core.

11. The composition of any one of Claims 8 to 10, wherein the therapeutic agent is an EGFR antibody.

12. The composition of Claim 11, wherein the therapeutic agent is cetuximab.

13. The composition of Claim 12, wherein the saccharide binding moiety is DBA.

14. The conjugate of any one of Claims 1-7 or the composition of any one of claims 8 to 13 for use in a method of treating cancer comprising administering said conjugate or composition to a subject diagnosed with cancer.

15. The conjugate or composition for use according to Claim 14, wherein:
a) the subject is diagnosed with a brain tumor; and/or
b) the subject has previously undergone surgical removal of a tumor or radiation therapy; and/or
c) the subject is administered the composition in combination with radiation therapy; and/or
d) the composition is administered orally, by injection, convection enhance delivery, or intracerebrally.
